# EUROPEAN PATENT SPECIFICATION

(11) **EP 4 066 869 B1**
(45) Date of publication and mention of the grant of the patent: **02.07.2025**
(21) Application number: 21770811.4
(22) Date of filing: 17.03.2021
(51) Int. Cl.: A61L 27/38, C12M 3/00, C12M 1/42, C12N 5/077, C12N 13/00

(54) **FIBROCARTILAGE PREPARATION METHOD USING TENSILE STIMULATION**
FASERKNORPELHERSTELLUNGSVERFAHREN UNTER VERWENDUNG VON ZUGSTIMULATION
PROCÉDÉ DE PRÉPARATION DE FIBROCARTILAGE UTILISANT UNE STIMULATION DE TRACTION

(30) Priority: 18.03.2020 KR 20200033429; 22.01.2021 KR 20210009580
(43) Date of publication of application: 05.10.2022
(73) Proprietor: UNIVERSITY-INDUSTRY COOPERATION GROUP OF KYUNG HEE UNIVERSITY, Yongin-si, Gyeonggi-do 17104 (KR)
(72) Inventor: LEE, Eunah, Seoul 05262 (KR); OH, Tong In, Seoul 01727 (KR)
(74) Representative: Isarpatent
(86) International application number: PCT/KR2021/003315
(87) International publication number: WO 2021/187898

(56) References cited:
- KR-B1- 101 085 193
- US-A1- 2010 119 577
- OUYANG XINLI, XIE YONGFANG, WANG GUOHUI: "Mechanical stimulation promotes the proliferation and the cartilage phenotype of mesenchymal stem cells and chondrocytes co-cultured in vitro", BIOMEDICINE & PHARMACOTHERAPY, vol. 117, 109146, 1 September 2019 (2019-09-01), FR, pages 1 - 7, XP055851236, ISSN: 0753-3322, DOI: 10.1016/j.biopha.2019.109146
- MORADI LIDA; VASEI MOHAMMAD; DEHGHAN MOHAMMAD M.; MAJIDI MOHAMMAD; FARZAD MOHAJERI SAEED; BONAKDAR SHAHIN: "Regeneration of meniscus tissue using adipose mesenchymal stem cells-chondrocytes co-culture on a hybrid scaffold: In vivo study", BIOMATERIALS, vol. 126, 20 February 2017 (2017-02-20), pages 18 - 30, XP029937167, ISSN: 0142-9612, DOI: 10.1016/j.biomaterials.2017.02.022
- LEE JENNIFER K., HUWE LE W., PASCHOS NIKOLAOS, ARYAEI ASHKAN, GEGG COURTNEY A., HU JERRY C., ATHANASIOU KYRIACOS A.: "Tension stimulation drives tissue formation in scaffold-free systems", NATURE MATERIALS, vol. 16, no. 8, 1 August 2017 (2017-08-01), pages 864 - 873, XP055851239, ISSN: 1476-1122, DOI: 10.1038/NMAT4917
- JANG S: "Effect of Mechanotransduction on Cartilaginous Matrix Production of Meniscal Chondrocytes", THESIS, 1 August 2020 (2020-08-01), Korea, pages 1 - 33, XP009530949

## Description

### TECHNICAL FIELD

The present invention relates to a fibrocartilage preparation method and fibrocartilage prepared by the method.

### BACKGROUND ART

Degenerative arthritis is a disease in which local degenerative changes appear while articular cartilage wears away, and also refers to osteoarthritis or osteoarthrosis. Degenerative arthritis is one type of chronic arthritis, and is a disease in which a degenerative change occurs in the articular cartilage subjected to weight-bearing, resulting in overgrowth of bone on the joint surface, and is often seen in middle-aged adults. In this disease, first, chondrocytes, which make up the constituent components of cartilage, lose their elasticity while deteriorating in function due to aging, and over time, the surface of cartilage becomes rough and various types of materials flow into the articular joint cavity surrounded by the joint membrane, so that inflammation occurs. Clinically, recurrent pain, joint stiffness, a progressive joint movement disorder in the joint, and the like appear.

The cause of degenerative changes in articular cartilage has not yet been elucidated, but it is known that an absolute decrease in the number of chondrocytes and an imbalance between cartilage matrix synthesis and degradation occurring in chondrocytes are one of the causes. Therefore, degenerative changes in articular cartilage reduce cartilage strength and cushioning ability due to the decomposition of the cartilage matrix.

As treatment for degenerative arthritis currently used clinically, drug treatment (analgesics, steroids, non-steroidal anti-inflammatory agents, and the like), chondroprotective agents (hyaluronic acid, glucosamine, chondroitin and the like), or is by a surgical treatment (arthroscopic surgery, wedge high tibial osteotomy, joint replacement, and the like). However, drug treatment has only an effect of non-specifically alleviating pain or an inflammatory response itself, and chondroprotective agents only serve to temporarily protect joints by simply supplying nutrients to chondrocytes or alleviating impact. In meniscus cartilage, which secures stability by fixing the shape and position according to joint movement between knee joint cartilage, a tear may occur due to partial damage, which causes pain, and in such cases, partial resection is performed to control the pain. In the case of a surgical procedure such as meniscus cartilage resection, partial or total-resection may ameliorate acute pain by primarily eliminating mechanical symptoms. However, there is a limitation in a sense that the progression of arthritis cannot be prevented since the surgical procedure increases the load on the articular cartilage in the long term and causes degenerative changes. If the meniscus tear was sutured instead of resection, although the indications are very limited, a considerable failure rate has been reported.

A cartilage treatment method using a tissue engineering technique is emerging to overcome the limitations of existing treatment methods for degenerative arthritis, and accordingly, there is a need for research and development on tissue engineering preparations in which a high degree of similarity to living body tissue is implemented by culturing transplantable tissue *in vitro.* The article OUYANG XINLI ET AL: "Mechanical stimulation promotes the proliferation and the cartilage phenotype of mesenchymal stem cells and chondrocytes co-cultured in vitro", BIOMEDICINE & PHARMACOTHERAPY, vol. 117, 1 September 2019 discloses a study where bone mesenchymal stem cells co-cultured with chondrocytes underwent cyclic sinusoidal dynamic tensile mechanical stimulation using a commercially available tension system, and the proliferation ability and cartilage phenotype of the cells were assayed. The article LEE JENNIFER K. ET AL: "Tension stimulation drives tissue formation in scaffold-free systems", NATURE MATERIALS, vol. 16, no. 8, 12 June 2017 describes a study on self-assembling neocartilage tissue, involving the use of tension stimulation alone or in combination with matrix remodelling and synthesis agents known to enhance tissue formation and organization.

### DESCRIPTION OF EMBODIMENTS

### TECHNICAL PROBLEM

The technical problem to be solved by the present invention is to provide a fibrocartilage preparation method.

Another technical problem to be solved by the present invention is to provide fibrocartilage prepared by the method.

### TECHNICAL SOLUTION

An aspect of the present invention relates to a method for preparing fibrocartilage, the method including: culturing under a tensile stimulus.

As used herein, the synovial cells refer to a group of cells present in the synovium, and may be used interchangeably with the same meaning as synovium-derived cells. The synovium is a tissue closest to the articular cartilage, and it has been reported that cells are recruited from the synovium to repair cartilage damage (Hunziker EB, Rosenberg LC, Repair of partial-thickness defects in articular cartilage: cell recruitment from the synovial membrane. The Journal of Bone and Joint Surgery. 1996 May;78(5):721-33), and furthermore, the synovium is the only tissue capable of producing hyaline cartilage in malignant conditions such as chondromatosis. This suggests that synovial cells may be utilized for the restoration of cartilage tissue.

In the present invention, chondrocytes are cells that differentiate from mesenchymal cells to form cartilage tissue, and include articular chondrocytes, costal chondrocytes, meniscal chondrocytes, and the like. It is known that the chondrocytes perform a function of synthesizing and secreting a cartilaginous matrix in the cartilage, and these cells gather to form fibrocartilage.

As used herein, mesenchymal stem cells (MSC) are a type of adult stem cells having adhesive properties, and are pluripotent stem cells capable of differentiating into chondrocytes, osteocytes, adipocytes, myocytes, and the like. Mesenchymal stem cells may be utilized for fibrocartilage formation using the ability of mesenchymal stem cells with abundant regenerative power to differentiate into chondrocytes.

In the present invention, fibrocartilage is classified according to its nature and shape, is a cartilage in which white fibrous tissue and cartilaginous tissue are mixed at various ratios, and refers to a site where ligaments or tendons attach to joints. The fibrocartilage is located in various forms such as the vertebral body, the pubic bone, the intervertebral disc between the knees, the meniscus cartilage, and the interpubic disc, is a hard fiber composed of collagen, is resistant to compression, but is well torn and arranged in a compactly tangled state, and is present in connection with hyaline cartilage or connective tissue.

In the present invention, the intervertebral disc is a fibrocartilage present between two vertebral bodies, and there are has 23 intervertebral discs in humans. The intervertebral disc is elastic and acts as a cushion that imparts flexibility to the spine and absorbs impact. When an excessive impact is applied or elasticity is lost due to aging, the fibrocartilage of the intervertebral disc may be crushed and protrude, and such a disease is called a hernia of intervertebral disc (often referred to as a disc).

In the present invention, as a cartilage commonly present in the knee joint, meniscus cartilage is a wedge-shaped cartilage that acts as a shock absorber between the femur and the tibia. Such meniscus cartilage serves to protect joints.

As used herein, the interpubic disc refers to a disc composed of fibrocartilage connecting between pubic bones in the pubic symphysis. Pubic bones are bones present in the hip joint, and the interpubic disc fibrocartilage is firmly bonded such that the two pubic bones are located at the midline.

Although scaffolds, synthetic polymers, biological matrices and the like have been used to form the fibrocartilage tissue in the related art, a method for forming a fibrocartilage tissue structure using only cells without a support has not been reported. This is because all the histological characteristics of a high expression level of type 1 collagen, uniaxial anisotropic tissue alignment, and cartilage-specific matrix expression should be implemented in order to expect that a cartilage tissue formed by *in vitro* culture has the same functionality as the cartilage tissue in the body.

In particular, when a tensile stimulus is applied, there is a problem in that it is difficult to form fibrocartilage tissue as the formation of cartilaginous matrix is suppressed.

In an exemplary embodiment of the present invention, it was confirmed that the nuclear localization tendency of YAP protein was increased and the expression level of Sox-9, which is a chondrocyte differentiation marker, was remarkably decreased after application of the tensile stimulus (FIGS. 2 and 3). Further, in an exemplary embodiment of the present invention, as a result of treatment with a material which suppresses the nuclear translocation of YAP protein, it was confirmed that the expression level of Sox-9 was again increased (FIG. 4).

From the aforementioned results, it was confirmed that when the tensile stimulus was applied, the nuclear translocation of YAP protein was promoted, and accordingly, the differentiation ability of chondrocytes was suppressed, and thus, the ability to form a cartilaginous matrix deteriorated.

Accordingly, in the present invention, it was confirmed that the suppression of cartilage formation was caused by the promotion of nuclear translocation of YAP protein by tensile stimulation, and a method capable of improving the ability to form a cartilaginous matrix by suppressing the nuclear translocation of YAP protein while maintaining the ability to form a fibrous matrix was developed by adjusting the degree and duration of a tensile stimulus to perform experiments under various culture conditions.

According to the invention, the tensile stimulus is applied for 3 to 10 days, more specifically for 7 to 10 days.

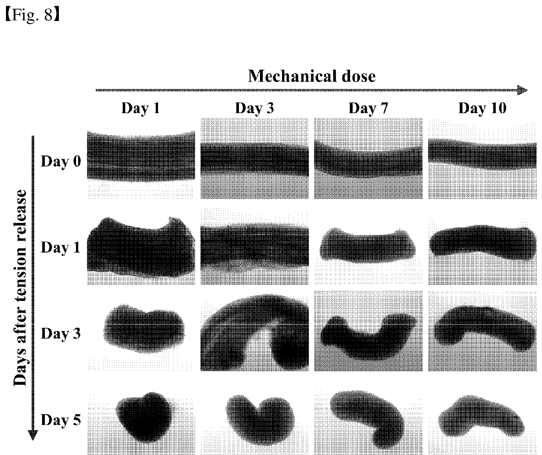

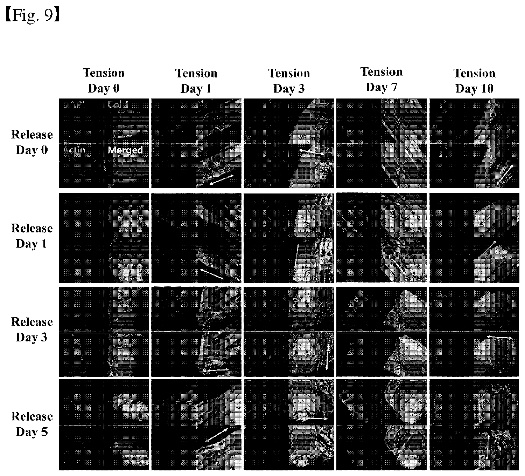

In an exemplary embodiment of the present invention, it was confirmed that when a tensile stimulus was applied for 3 days or more, the form of cartilage was formed, and alignment was maintained even though the tensile stimulus was released, and it was confirmed that when the tensile stimulus was applied for 7 days or more, the form and uniaxial anisotropic tissue alignment were maintained for 5 days or more even though the tensile stimulus was released (FIGS. 8 and 9).

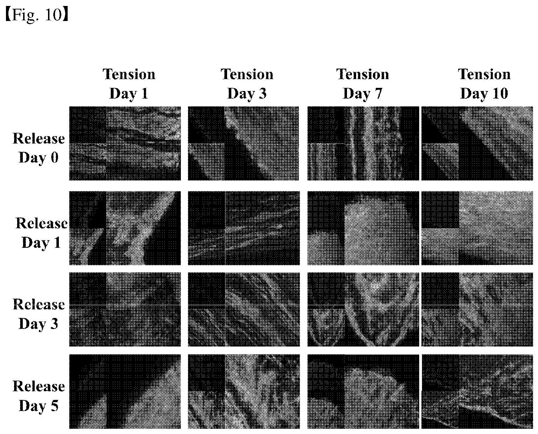

In addition, it was confirmed that it is effective to apply a tensile stimulus within 10 days in order to prevent the irreversible nuclear localization tendency of YAP protein (FIG. 10).

That is, in the present invention, it was confirmed that by applying tensile stimulus, fibrocartilage was formed and the form was maintained, and furthermore, it was confirmed that the tendency of YAP protein to localize in the nucleus could be suppressed by adjusting the tensile stimulus duration, so that fibrocartilage may be effectively formed and prepared without a special support such as a scaffold.

Specifically, the tensile stimulus may be applied with a force of 5% to 20%, more specifically, 5% to 15%, and most specifically, 10% to 15%.

Further, specifically, after chondrocytes were cultured under the tensile stimulation, a step of removing the tensile stimulus and culturing the chondrocytes for 10 to 28 days may be additionally included. According to the invention chondrocytes are cultured in a cartilage differentiation medium for 14 to 21 days.

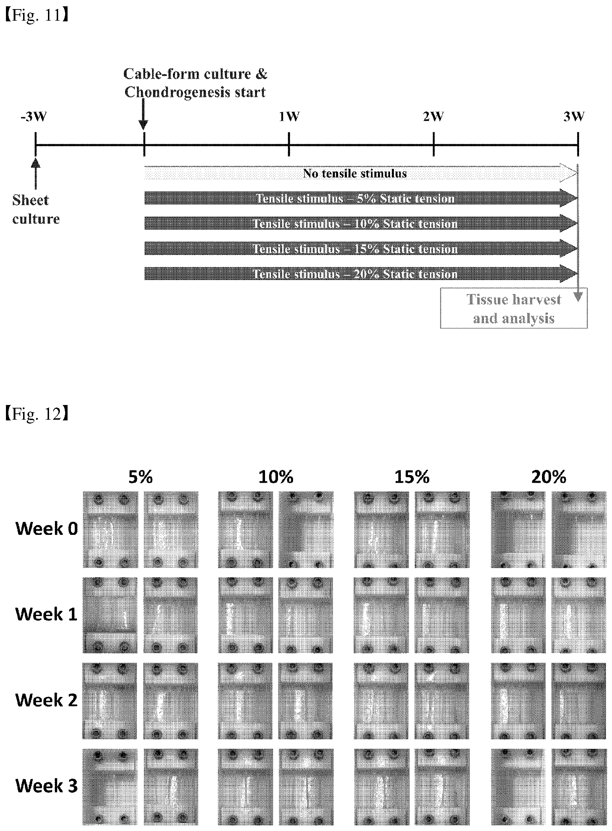

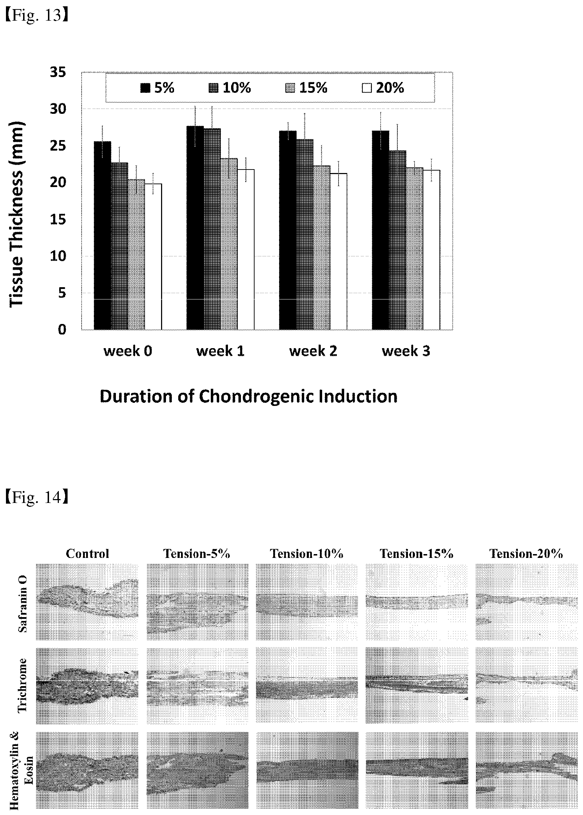

In an exemplary embodiment of the present invention, it was confirmed that when chondrocytes were cultured in a state where a tensile stimulus of 5 to 20% was applied, the thickness was increased as tissue was formed (FIGS. 12 and 13). In addition, it was confirmed that when a tensile stimulus of 5 to 10% was applied, the thickness of tissue was increased, and consistent tissue alignment was more apparent throughout the tissue, and in the case of a tensile stimulus of 15% or more, a high level of tissue alignment was exhibited along with an increase in tissue formation in consideration of an increase in the length of the entire tissue (FIG. 14).

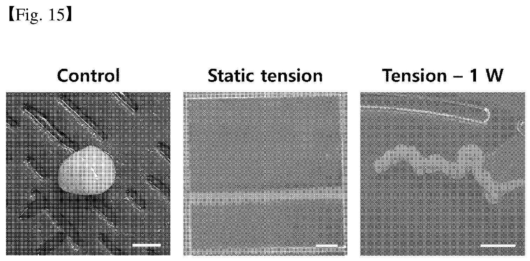

In an exemplary embodiment of the present invention, as a result of culturing chondrocytes in a cartilage differentiation medium for 3 weeks in a state where the tensile stimulus was applied and then released, it was confirmed that fibrocartilage was formed. In contrast, it was confirmed that when the tensile stimulus was continuously applied for 3 weeks, fibrocartilage was not formed (FIG. 15).

The above results suggest that when the tensile stimulus is sustained for a certain period of time, the nuclear translocation of YAP protein appears continuously, the ability to form a cartilaginous matrix is suppressed, and it may be difficult to form fibrocartilage, but when chondrocytes are cultured by temporarily applying the stimulation for about 5 to 10 days, and then releasing the stimulation, the nuclear translocation of YAP protein can be prevented while maintaining tissue alignment.

The method may reduce the suppression of chondrocyte differentiation. That is, the ability to form a cartilaginous matrix may be improved.

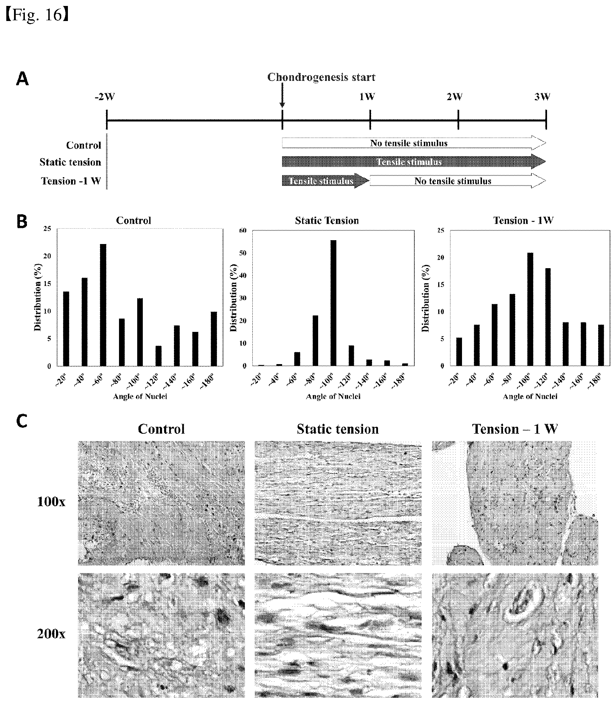

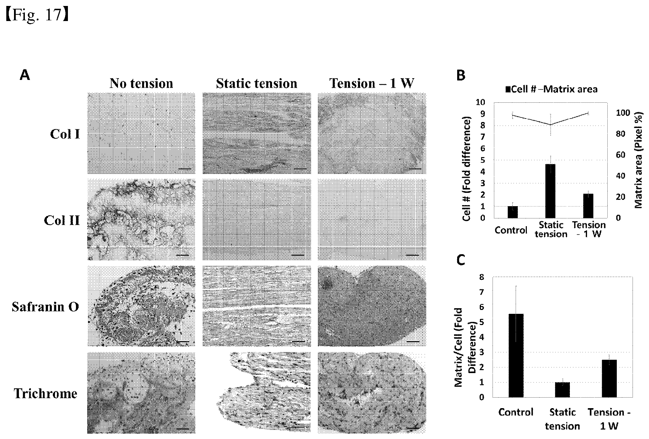

In an exemplary embodiment of the present invention, as a result of applying a tensile stimulus, and then releasing the tensile stimulus and culturing chondrocytes in a chondrocyte differentiation medium, it was confirmed that a high level of tissue alignment was maintained (FIG. 16), and it was confirmed that both the synthesis amount of type 1 collagen and the synthesis amount of type 2 collagen were increased, and thus both a fibrous matrix and a cartilaginous matrix could be formed (FIG. 17).

According to the above results, by adjusting the degree and duration of a tensile stimulus to culture chondrocytes, the present invention may solve a problem of chondrocyte differentiation suppression caused by tensile stimulation in the related art, and may form a fibrocartilage tissue with excellent ability to form a fibrous matrix and a cartilaginous matrix.

Specifically, the fibrocartilage tissue may be one or more selected from the group consisting of the intervertebral disc, the interpubic disc and the meniscus cartilage, but is not limited thereto.

Furthermore, specifically, the chondrocytes may be any one selected from the group consisting of mesenchymal stem cells, articular chondrocytes, costal chondrocytes, and meniscal chondrocytes, but are not limited thereto, and any chondrocytes that can be utilized for the formation of fibrocartilage tissue may be included.

Another aspect of the present invention relates to fibrocartilage prepared by the method.

The description of the fibrocartilage and the fibrocartilage preparation method is the same as described above.

The fibrocartilage may be selected from the group consisting of the intervertebral disc, the interpubic disc and the meniscus cartilage, but is not limited thereto.

In an exemplary embodiment of the present invention, it was confirmed that a fibrocartilage tissue prepared by the method has both excellent tissue alignment and excellent ability to form a cartilaginous matrix (FIGS. 8 to 13).

### ADVANTAGEOUS EFFECTS OF INVENTION

Since the fibrocartilage preparation method of the present invention can form fibrocartilage tissue having high bio-similarity through *in vitro* culture, the fibrocartilage tissue can be utilized for tissue regeneration and transplantation for fibrocartilage reconstruction.

The effect of the present invention is not limited to the aforementioned effects, and it should be understood to include all possible effects deduced from the configuration of the invention described in the the claims of the present invention.

### BRIEF DESCRIPTION OF THE DRAWINGS

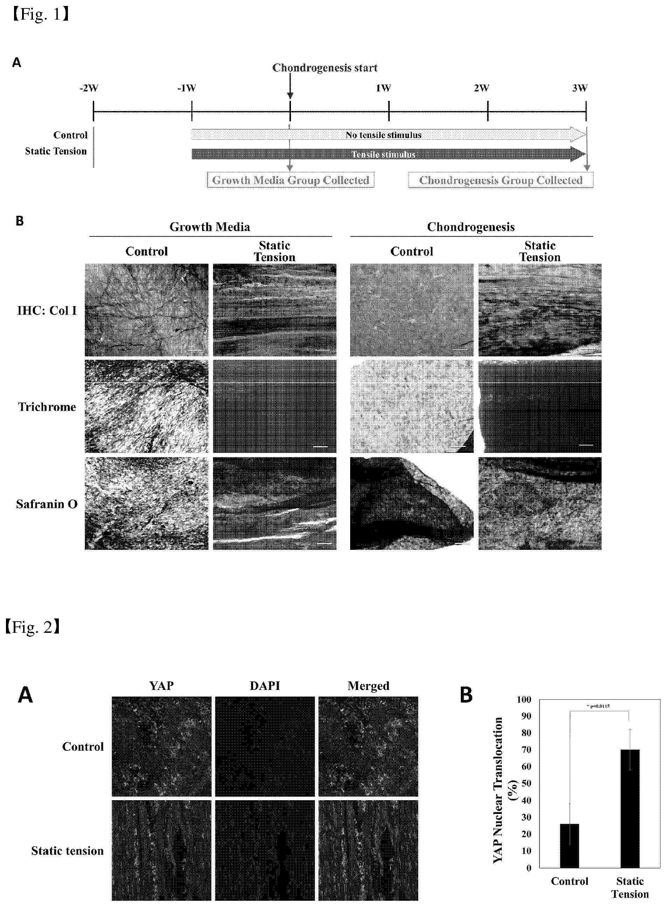

FIG. 1 shows the characteristics of the tissues formed by culturing a treatment group to which a tensile stimulus is applied and a control to which the tensile stimulus was not applied.
FIG. 2 shows the results of confirming the nuclear localization tendency of YAP protein after application of a tensile stimulus.

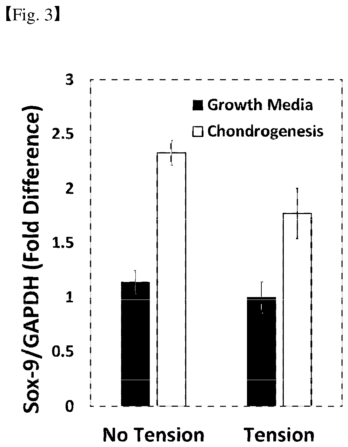

FIG. 3 shows the results of confirming the expression level of Sox-9 after application of a tensile stimulus.

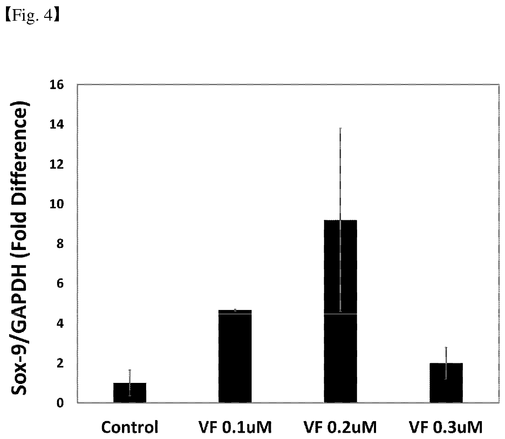

FIG. 4 shows the results of confirming whether the expression of Sox-9 is increased by the treatment with an inhibitor of nuclear translocation of YAP protein.

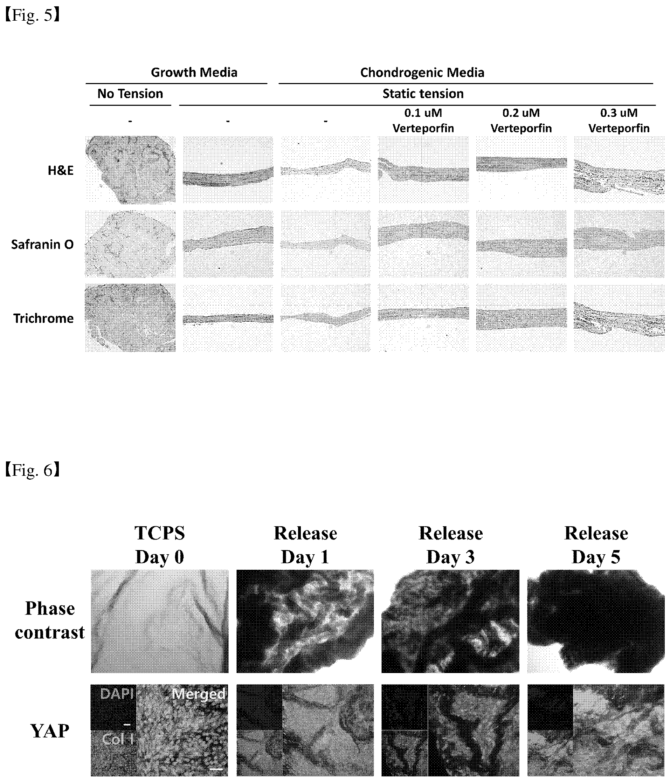

FIG. 5 shows the results of confirming the ability to form a cartilage matrix when a group to which a tensile stimulus is applied is treated with an inhibitor of the nuclear translocation of YAP protein.
FIG. 6 shows the results of confirming whether YAP protein translocates into the cytoplasm according to surface hardness.

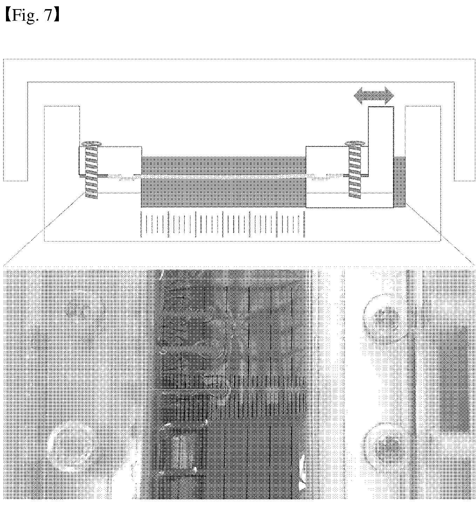

FIG. 7 shows a static tension chamber structure.
FIG. 8 shows the results of confirming whether tissue alignment is maintained after a tensile stimulus is applied and then the tension is released.
FIG. 9 shows the results of fluorescent staining of the actin cytoskeleton and type 1 collagen after a tensile stimulus is applied and then the tension is released.
FIG. 10 shows the results of confirming the nuclear localization tendency of YAP protein after a tensile stimulus is applied and then the tension is released.
FIG. 11 is a schematic view illustrating the duration and conditions of culturing while applying a tensile stimulus of 5% to 20%.
FIG. 12 shows the results of confirming the change in tissue thickness for each culture week by comparing macroscopic photographs of tissues cultured by applying tensile stimuli of 5%, 10%, 15%, and 20%.
FIG. 13 shows the results of confirming the change in tissue thickness with time under each condition for application of a tensile stimulus.
FIG. 14 shows the results of performing safranin O, trichrome, and H&E staining to confirm the matrix characteristics of the tissues obtained 3 weeks after the induction of cartilage differentiation.
FIG. 15 shows the results of confirming whether fibrocartilage can be formed when a tensile stimulus is temporarily (7 days) applied, continuously applied, or not applied.
FIG. 16 shows the results of confirming the degree of tissue alignment maintenance when a tensile stimulus is temporarily (7 days) applied, continuously applied, or not applied.
FIG. 17 shows the results of confirming whether a fibrous matrix and a cartilaginous matrix are formed when a tensile stimulus is temporarily (7 days) applied, continuously applied, or not applied.

### DETAILED DESCRIPTION OF EXEMPLARY EMBODIMENTS

Hereinafter, the present invention will be described in detail through the Examples. However, the following Examples are only for exemplifying the present invention, and the present invention is not limited by the following Examples.

### Example 1. Confirmation of tissue alignment and whether a cartilage matrix formation by application of tensile stimulus

### 1-1. Confirmation of tissue alignment and whether a cartilage matrix formation by application of tensile stimulus

After chondrocytes obtained from meniscus cartilage, which is fibrocartilage, were cultured in the form of a sheet, the characteristics of a tissue formed by sequentially culturing a treatment group to which a static tension of 10% was applied and a control to which the tension was not applied in a growth medium (alpha-MEM including 2 mM L-glutamine, 10⁻⁴ M ascorbic acid, 10⁻⁸ M dexamethasone, 1 ng/ml rhFGF-2, and 5% fetal bovine serum) and a cartilage differentiation medium (DMEM including 1 M proline, 0.3 mM ascorbic acid, 10⁻⁷ M dexamethansone, 10 ng/ml TGF-3, and 1x IT+3) were confirmed.

First, in order to confirm the arrangement and synthesis amount of type 1 collagen, immunostaining using an antibody against type 1 collagen (Chemicon, product number AB755P) was performed, and trichrome staining was performed with reference to the methods described in L G Luna literature (Histopathologic Methods and Color Atlas of Special Stains and Tissue Artifacts, Johnson Printers, Downers Grove, IL P151-152 c 1992).

Further, after the cells cultured in the medium were fixed using 4% paraformaldehyde, the fixed cells were embedded in paraffin were cut into sections, and then de-paraffinized using xylene. Thereafter, nuclear staining was performed for 5 minutes using Weigert's hematoxylin, and the cells were washed with distilled water for 10 minutes. After 10 minutes, the cells were rinsed using a 70% alcohol solution containing 1% HCl and then stained with 0.02% aqueous fast green for 5 minutes. After being stained with fast green, the cells were washed with 1% acetic acid and stained using 0.1% aqueous safranin O for 5 minutes. After staining with safranin O, the sample was immersed in alcohol 2 to 3 times, and then put into xylene for mounting.

As a result, it was confirmed that when a tensile stimulus was applied, a high level of tissue alignment was induced, and the degree of arrangement and the synthesis amount of type 1 collagen, which is a fibrous matrix, were both increased, and meanwhile, it was confirmed that no formation of cartilaginous matrix was observed in the group treated with the tensile stimulus (FIG. 1).

### 1-2. Confirmation of signal factor for suppressing cartilaginous matrix formation

In order to confirm whether the tensile stimulus induced the nuclear translocation phenomenon of the YAP protein present in the cytoplasm, immunofluorescence (IF) method was performed on the treatment group cultured in 1-1 above.

Specifically, 3.7% paraformaldehyde was added to the cells and the cells were fixed at room temperature for 20 minutes. Thereafter, the cells were washed 3 times with PBS and treated with 0.1% Triton-X100 for 15 minutes to increase the permeability of the cells. After the cells were again washed 3 times with PBS, the cells were treated with 10% fetal bovine serum (FBS) for 1 hour, an anti-YAP antibody was diluted with 10% FBS, reacted at room temperature for 2 hours, and washed 3 times with 1% FBS for 10 minutes, and then a secondary antibody was diluted with 10% FBS, reacted in the dark for 2 hours, and washed 3 times with 1% FBS for 10 minutes each. In this case, during the second washing, 300 ng/ml DAPI was put into 1% FBS. Next, an encapsulation solution was dropped on a slide glass, and then the slide glass was covered with a cover glass. The surrounding encapsulation solution was removed, the cover glass was covered with enamel, and then observed under a microscope.

In addition, semi-quantitative PCR (30 cycles optimal) was performed to investigate the expression level of a cartilage differentiation marker Sox-9. Specifically, the primer sequences of SEQ ID NOS: 1 and 2 were used, and the expression level of glyceraldehyde-3-phosphate dehydrogenase (GAPDH) was used for the normalization of gene expression values.

**[Table 1]**

| SEQ ID NO | Primer | Sequence (5'-3') |
|---|---|---|
| 1 | Sox-9 forward | CTCCGACACCGAGAATACAC |
| 2 | Sox-9 reverse | CCATTCTTCACCGACTTCCT |
| 3 | GAPDH forward | TCACCATCTTCCAGGAGCGA |
| 4 | GAPDH reverse | CACAATGCCGAAGTGGTCGT |

As a result, it was confirmed that the amount of YAP protein translocated to the nucleus was remarkably increased during culture while maintaining the tensile stimulus for 3 weeks (FIG. 2).

Furthermore, it was confirmed that a basal level of Sox-9 expression appeared under a growth medium condition, and the Sox-9 expression was increased in both the control and the treatment group under a cartilage differentiation medium condition. However, under the cartilage differentiation condition, a decrease in Sox-9 expression was observed in the treatment group compared with the control (FIG. 3).

The above results suggest that the localization of YAP protein in the nucleus by a tensile stimulus affects the regulation of Sox-9 expression, resulting in a deterioration in the shape of a cartilaginous matrix.

### 1-3. Confirmation of ability to form cartilage during treatment with inhibitor of YAP protein nuclear translocation

It was confirmed whether the expression of Sox-9, which is a cartilage differentiation marker, was increased during treatment with verteporfin, which suppresses the translocation of YAP protein to the nucleus. The Sox-9 expression level was measured in the same manner as in 1-2.

Further, a cartilage tissue obtained after the treatment with verteporfin was embedded in paraffin, sectioned to a thickness of 5 to 7 µm, dried, and then subjected to hematoxylin & eosin (H&E) staining. The stained tissue was observed under a high resolution optical microscope.

As a result, it was confirmed that the expression of the cartilage differentiation marker Sox-9 was remarkably increased during the treatment with verteporfin, suggesting that the cartilage differentiation ability can be highly exhibited as the translocation of the YAP protein to the nucleus is suppressed. Meanwhile, when the treatment group to which the tensile stimulus was applied was treated with verteporfin, the overall matrix forming ability of the tissue was increased, whereas the formation of the fibrous matrix showed a decreasing pattern, suggesting that suppressing the translocation of YAP protein alone does not establish effective fibrocartilage formation conditions.

Through the above results, it was confirmed that when a tensile stimulus is applied, the fibrous matrix could be formed in a tissue anisotropy direction, but cartilage matrix formation was suppressed by promoting the translocation of YAP protein to the nucleus, and thus, it was difficult to form a fibrocartilage tissue.

### Example 2. Search for tensile application conditions under which tissue anisotropy is maintained

### 2-1. Confirmation of conditions for suppressing translocation of YAP protein to nucleus

Conditions for eliminating the effect of suppressing cartilage differentiation according to the nuclear localization tendency of YAP protein were confirmed. First, after a cell sheet was detached from a plastic culture dish, only cell-cell contact was allowed to be maintained. One day later, the intracellular distribution of YAP protein was confirmed in the same manner as in Example 1-2.

As a result, it was confirmed that when the stimulus that induced the translocation of YAP protein disappeared, the short-term localization of YAP was adjusted, and thus the YAP was distributed in the cytoplasm (FIG. 6). This suggests that the suppression of cartilage differentiation by YAP can be prevented.

### 2-2. Confirmation of whether tissue alignment is maintained when tensile stimulus is applied and then tension is released

Based on the experimental results of 2-1 above, it was confirmed whether tissue alignment was maintained even after a tensile stimulus was applied for 1 to 10 days, and then the tension was released.

First, chondrocytes cultured in the form of a sheet were rolled up longitudinally to form a cell cable, and a tensile stimulus of 10% was applied for 1 to 10 days using the static tension chamber designed in FIG. 7, and it was confirmed whether tissue alignment was maintained after 1, 3, 7, and 10 days. After the tensile stimulus was applied, the arrangement form of an actin cytoskeleton using phalloidin staining was confirmed in order to carefully evaluate the degree of tissue alignment maintenance, and the arrangement form of the extracellular matrix was confirmed by fluorescently staining type 1 collagen in the same manner as in 1-1 above.

As a result of macroscopic observation of the tissue form, it was confirmed that when the tensile stimulus was applied for 3 days or more, the form was produced and tissue alignment was maintained, and when the tensile stimulus was applied for 7 days or more, the form was maintained for 5 days or more even after the tensile stimulus was released (FIG. 8).

As a result of confirmation using immunofluorescent staining to determine whether tissue alignment is maintained based on the arrangement form of the cytoskeleton and extracellular matrix, it was confirmed that when a tensile stimulus was applied for 3 days, and then the tensile stimulus was released, the alignment of the cytoskeleton and extracellular matrix became disordered after 5 days, whereas when the tensile stimulus was applied for 7 days or more, and then the tensile stimulus was released, the uniaxial anisotropic tissue alignment was maintained for 5 days (FIG. 9).

### 2-3. Confirmation of nuclear localization tendency of YAP protein when tensile stimulus is applied and then tension is released

Based on the 2-1 experiment, an experiment was conducted to confirm whether YAP protein was translocated to the nucleus when a tensile stimulus was applied for 1, 3, 7, and 10 days, and then the tension was released. The experiment was performed in the same manner as in 1-2 above.

As a result, it was confirmed that the nuclear localization tendency of YAP protein was remarkably reduced 24 hours after the release of the tensile stimulus under all the tensile stimulus conditions, and it was confirmed that the temporary tensile stimulus applied in the form of a cell cable did not induce irreversible changes in regard to the translocation of YAP protein to the nucleus, and thus, the effect of suppressing cartilage differentiation may not be sustained (FIG. 10).

From the above results, it was confirmed that the duration of tensile stimulus application needs to exceed at least 3 days in order to maintain tissue alignment at the initial stage (5 to 7 days) of the cartilaginous matrix formation, while a tensile stimulus needs to be applied within a maximum of 10 days in order to prevent the irreversible nuclear localization tendency of YAP protein.

### Example 3. Confirmation of tissue formation according to application of tensile structure

Based on the experimental results of Example 2, a cartilage tissue to be formed while being cultured using the cartilage differentiation medium for 3 weeks was confirmed in a state where a tensile stimulus of 5 to 20% was applied. The duration and conditions of culturing while applying a tensile stimulus of 5 to 20% are as illustrated in FIG. 11.

The thickness of the tissue formed for 3 weeks was observed with the naked eye, and as a result, it was confirmed that the growth of the tissue was increased over time throughout the entire tensile stimulus range of 5 to 20% (FIGS. 12 and 13). In addition, it was confirmed that when a tensile stimulus of 5 to 10% was applied, the thickness of tissue was increased, and consistent tissue alignment was more apparent throughout the tissue, and in the case of a tensile stimulus of 15% or more, a high level of tissue alignment was exhibited along with an increase in tissue formation in consideration of an increase in the length of the entire tissue (FIG. 14).

Furthermore, in order to induce cartilage differentiation by applying the tensile stimulus as described above and confirm the characteristics of a cartilage tissue obtained after 3 weeks, the tissue was observed after being stained with safranin O, trichrome, and H&E. As a result, it was confirmed that a tissue with uniaxial anisotropic tissue alignment was formed by a tensile stimulus of 5 to 20%, and the alignment of the tissue appeared more consistently, particularly in a tensile stimulus range of 5 to 15%.

### Example 4. Confirmation of formation of cartilage tissue in which anisotropy is implemented

### 4-1. Confirmation of fibrocartilage formation after tensile stimulus

Based on the experimental results of Example 2, it was confirmed whether fibrocartilage could be formed after a tensile stimulus of 10% was applied for 7 days.

First, fibrocartilage tissue was formed by culturing a treatment group in which a tensile stimulus of 10% was applied to chondrocytes for 7 days in a cartilage differentiation medium for 3 weeks. Thereafter, it was observed under a microscope whether tissue formation occurred.

As a result, it was confirmed that the cartilage tissue was formed when the cells were cultured for 2 to 3 weeks by applying a tensile stimulus of 10% for 7 days, and then releasing tension (FIG. 15).

### 4-2. Confirmation of maintenance of tissue alignment after tensile stimulus

The degree of tissue alignment maintenance after cartilage differentiation was evaluated by investigating an angle exhibited by the nuclei, and thereafter, it was confirmed under a microscope whether alignment was maintained.

As a result, it was confirmed that when the tensile stimulus was not applied, alignment in a specific direction did not appear, but a high level of alignment was maintained in a group treated with the tensile stimulus for 7 days or a group continuously treated with the tensile stimulus for 3 weeks (FIG. 16).

### 4-3. Confirmation of cartilage differentiation characteristics after tensile stimulus

After a tensile stimulus of 10% was applied for 7 days, immunostaining and histological staining were performed on a fibrous matrix and a cartilaginous matrix. The experimental method was performed in the same manner as in Example 1-1, and for staining of type 2 collagen, immunohistochemical staining was performed using an antibody against type 2 collagen (Abcam, product number AB53047).

As a result, it was confirmed that in a group cultured in a state in which a tensile stimulus was applied for 7 days, and then released, the formation of both type 1 collagen and the cartilaginous matrix appeared, and the area of extracellular matrix formed per cell was shown to be remarkably high compared to the tensile stimulus maintenance group. On the other hand, in the control to which the tensile stimulus was not applied, the degree of type 1 collagen formation appeared very low, and when the tensile stimulus was continuously applied for 3 weeks, almost no cartilaginous matrix formation appeared.

Through the above results, it can be seen that when chondrocytes are cultured under a tensile stimulus, and then cultured in a tension-released state, the ability to form both a fibrous matrix and a cartilaginous matrix is excellent, suggesting that by applying the method of the present invention to culture chondrocytes, it is possible to form a tissue capable of simultaneously exhibiting tensile strength and compressive strength that can withstand the load applied in the joint.

The above-described description of the present invention is provided for illustrative purposes.

Therefore, it should be understood that the above-described embodiments are only exemplary in all aspects and are not restrictive. For example, each constituent element which is described as a singular form may be implemented in a distributed form, and similarly, constituent elements which are described as being distributed may be implemented in a combined form.

The scope of the present invention is represented by the following claims.

## Claims

1. A method for preparing fibrocartilage, the method comprising: culturing chondrocytes under a tensile stimulus,
wherein the tensile stimulus is applied for 3 days to 10 days,
wherein the method further comprises a step of culturing the cells for an additional 14 to 21 days in a cartilage differentiation medium after removing the tensile stimulus,
wherein the fibrocartilage is formed and prepared without a scaffold.

2. The method of claim 1, wherein the applied tensile stimulus is 5% to 20%.

3. The method of claim 1, wherein the fibrocartilage is one or more selected from the group consisting of the intervertebral disc, the interpubic disc and the meniscus cartilage.

4. The method of claim 1, wherein the cartilage cells are any one selected from the group consisting of articular chondrocytes, costal chondrocytes, and meniscal chondrocytes.

5. Fibrocartilage prepared by the method of claim 1.

## Patentansprüche

1. Verfahren zur Herstellung von Faserknorpel, wobei das Verfahren Folgendes umfasst: Kultivieren von Chondrozyten unter einem Zugreiz,
wobei der Zugreiz 3 Tage bis 10 Tage lang ausgeübt wird,
wobei das Verfahren ferner einen Schritt zum Kultivieren der Zellen für weitere 14 bis 21 Tage nach Absetzen des Zugreizes in einem Knorpeldifferenzierungsmedium umfasst,
wobei der Faserknorpel ohne Gerüst ausgebildet und hergestellt wird.

2. Verfahren nach Anspruch 1, wobei der ausgeübte Zugreiz 5 % bis 20 % beträgt.

3. Verfahren nach Anspruch 1, wobei der Faserknorpel ein oder mehrere Knorpel ist, die aus der Gruppe ausgewählt sind bestehend aus dem Knorpel der Bandscheibe, der Schambeinfuge und des Meniskus.

4. Verfahren nach Anspruch 1, wobei die Knorpelzellen beliebige Zellen sind, die aus der Gruppe ausgewählt sind bestehend aus Chondrozyten in Gelenken, Chondrozyten der Rippen und Chondrozyten der Menisken.

5. Faserknorpel, hergestellt mit dem Verfahren nach Anspruch 1.

## Revendications

1. Procédé de préparation de fibrocartilage, le procédé comprenant : la culture de chondrocytes sous un stimulus de traction,
le stimulus de traction étant appliqué durant 3 jours à 10 jours,
le procédé comprenant en outre une étape de culture des cellules durant 14 à 21 jours supplémentaires dans un milieu de différenciation de cartilage après l'enlèvement du stimulus de traction,
le fibrocartilage étant formé et préparé sans un échafaud.

2. Procédé selon la revendication 1, dans lequel le stimulus de traction appliqué est de 5 % à 20 %.

3. Procédé selon la revendication 1, dans lequel le fibrocartilage est un ou plusieurs choisis dans le groupe constitué par le cartilage du disque intervertébral, du disque interpubique et du ménisque.

4. Procédé selon la revendication 1, dans lequel les cellules de cartilage sont l'une quelconque choisie dans le groupe constitué par les chondrocytes articulaires, les chondrocytes costaux et les chondrocytes du ménisque.

5. Fibrocartilage préparé par le procédé selon la revendication 1.
